# EUROPEAN PATENT SPECIFICATION

(11) **EP 2 671 591 B1**
(45) Date of publication and mention of the grant of the patent: **09.08.2017**
(21) Application number: 12171326.7
(22) Date of filing: 08.06.2012
(51) Int. Cl.: A61K 38/00, A61K 9/16, A61K 9/19

(54) **Process for the preparation of short-chain peptide powders**
Verfahren zur Herstellung kurzkettiger Peptidpulver
Procédé pour la préparation de poudres de peptides à chaîne courte

(43) Date of publication of application: 11.12.2013
(73) Proprietor: Corden Pharma Brussels, 1120 Brussels (BE)
(72) Inventor: Tambuyser, Thierry, B-1120 Brussels (BE); Wilmet, Vincent, B-1300 Wavre (BE); Devenyns, Johan, B-1730 Asse (BE)
(74) Representative: Office Kirkpatrick

(56) References cited:
- WO-A2-03/043574
- YU Z ET AL: "Preparation and characterization of microparticles containing peptide produced by a novel process: spray freezing into liquid", EUROPEAN JOURNAL OF PHARMACEUTICS AND BIOPHARMACEUTICS, ELSEVIER SCIENCE PUBLISHERS B.V., AMSTERDAM, NL, vol. 54, no. 2, 1 September 2002 (2002-09-01), pages 221-228, XP004377367, ISSN: 0939-6411, DOI: 10.1016/S0939-6411(02)00050-4
- YU Z ET AL: "Spray freezing into liquid nitrogen for highly stable protein nanostructured microparticles", EUROPEAN JOURNAL OF PHARMACEUTICS AND BIOPHARMACEUTICS, ELSEVIER SCIENCE PUBLISHERS B.V., AMSTERDAM, NL, vol. 58, no. 3, 1 November 2004 (2004-11-01), pages 529-537, XP004586433, ISSN: 0939-6411, DOI: 10.1016/J.EJPB.2004.04.018
- YU Z ET AL: "Spray freezing into liquid versus spray-freeze drying: Influence of atomization on protein aggregation and biological activity", EUROPEAN JOURNAL OF PHARMACEUTICAL SCIENCES, ELSEVIER, AMSTERDAM, NL, vol. 27, no. 1, 1 January 2006 (2006-01-01), pages 9-18, XP027996502, ISSN: 0928-0987 [retrieved on 2006-01-01]
- W. THOMAS LEACH ET AL: "Uniform encapsulation of stable protein nanoparticles produced by spray freezing for the reduction of burst release", JOURNAL OF PHARMACEUTICAL SCIENCES, vol. 94, no. 1, 1 January 2004 (2004-01-01), pages 56-69, XP055040332, ISSN: 0022-3549, DOI: 10.1002/jps.20209
- METTE THING ET AL: "Thiol-disulfide interchange in the tocinoic acid/glutathione system during freezing and drying", JOURNAL OF PHARMACEUTICAL SCIENCES, vol. 99, no. 12, 17 May 2010 (2010-05-17), pages 4849-4856, XP055040334, ISSN: 0022-3549, DOI: 10.1002/jps.22206
- Berg JM, Tymoczko JL, Stryer L.: "Section 3.2: Primary Structure: Amino Acids Are Linked by Peptide Bonds to Form Polypeptide Chains", Biochemistry. 5th edition , 2002, XP002684827, New York Retrieved from the Internet: URL:http://www.ncbi.nlm.nih.gov/books/NBK2 2364/ [retrieved on 2012-10-12]
- Sunday A. Shoyele: "Chapter 7: Engineering Protein Particles for Pulmonary Drug Delivery" In: Kewal K. Jain: "Drug delivery systems (Methods in molecular biology)", 2008, Springer Science + Business Media, LLC, XP002684828, ISBN: 978-1-58829-891-1 vol. 437, page 153, * page 153 *

## Description

### Field of the invention

The present invention relates to a process or method for preparing short-chain peptide powders. The process comprises subjecting the corresponding short-chain peptide solutions to two consecutive operations : a flash-freezing step a., followed by a lyophilization step b. of the pellets resulting from step a.

### Background of the invention

European patent application EP 2 062 909 A1, published May 27, 2009, discloses a process for the production and purification of short-chain peptides.

Paragraph 74 of said application discloses that the short-chain peptide produced according to the method as claimed therein can be separated from a solution for example by spray-drying, filtration or decantation and dried before optionally being submitted to further processing steps such as combining with other ingredients, lyophilization, packaging and/or storage. Paragraph 97 of said application describes a specific implementation of the above treatment processes. The solution of the short-chain peptide obtained in example 8 of said specification was concentrated under vacuum and subsequently lyophilized in freeze-drying trays. To this end, the peptide solution was placed in a freeze-dryer for lyophilization. The freeze-drying trays were cooled to -40°C for 3 h. Afterwards, the temperature was raised to 20°C under vacuum (0.22 mbar) for 17 h. Afterwards, the temperature was maintained at 20°C for 4 h with the vacuum adjusted to 0.02 mbar. This yielded the short-chain peptide in the form of a white powder. The above description is an example of the prior art technique applied so far to this kind of short-chain peptides. This comprises conventional freezing of the peptide solution as an overall global batch, followed by lyophilization. This prior art process is characterized by a long process time required to freeze the peptide solution.

With regards to proteins, it has been reported that they tend to aggregate when the prior art technique of traditional freezing and lyophilization process is applied. Apparently, the aggregation tendency renders control of their morphology difficult, if not impossible and gives rise to various quality defects of the proteins so treated. It has also been reported that their potency is substantially reduced and that they have been implicated in life-threatening immunogenic reactions to protein drugs. Controlled morphology of peptides is indeed a key condition for the therapeutic application of an API peptide.

Furthermore, heterogeneousness in the structure and chemical content variations in the protein products obtained with traditional freeze-drying operations appear often and clearly should be avoided. Reproducibility of the process and homogeneousness of the protein powders are indeed critical factors for their applications.

With regards to proteins, flash freezing has been proposed as an effective remedy against aggregation, in particular by spraying small droplets of a protein solution into liquid nitrogen, and removing the frozen pellets from the liquid nitrogen vessel for subsequent lyophilization. A flash-freezing process for proteins is disclosed in the following patent publication : WO2009/002874. However, it was reported in WO2009/002874 that the complex geometry of the turbulent spray in the liquid nitrogen appears to be difficult to control and monitor, and therefore, discourages the use of such flash freezing technique in a liquid nitrogen bath as a means to solve the aggregation issue of proteins.

The inclusion of excipients is proposed as a solution to avoid such aggregation, apart from flash freezing the proteins in liquid nitrogen, prior to lyophilization. These excipients modify the physical properties of the proteins, thereby avoiding such aggregation. Examples of such excipients are carbohydrates, such as sucrose or trehalose, and Tween-20. Additional prior art includes YU Z ET AL: "Preparation and characterization of microparticles containing peptide produced by a novel process: spray freezing into liquid", EUROPEAN JOURNAL OF PHARMACEUTICS AND BIOPHARMACEUTICS, ELSEVIER SCIENCE PUBLISHERS B.V., AMSTERDAM, NL, vol. 54, no. 2, 1 September 2002 (2002-09-01), pages 221-228 and it describes the use of a preparation method for protein nanostructured microparticles, wherein the microparticles are produced by a spray freezing into liquid process.

### Aim of the invention

Purpose of the invention is to solve the quality problems regarding the morphology of the short-chain peptides as described above, e.g. with regards to aggregation. It is also an object of the invention to provide an efficient and cost-effective process for the manufacture of short-chain peptide powders starting from the corresponding short-chain peptide solutions. Another object of the invention is to provide a process to reproducibly and without loss of quality of the short-chain peptides attain the overall specifications of such peptide powders. Yet another object of the invention is to provide a process for the manufacture of short-chain peptide powders with an improved content of the short-chain peptide, an improved content of residual solvent, such as e.g. water, an improved content of residual counter-ion, in particular acetate and trifluoroacetate, an improved content of residual solvent aid, such as e.g. acetonitrile or terbutanol and/or an improved content of residual acids. A further aim is to provide frozen pellets comprising short-chain peptides with improved handling properties. Yet another aim is to provide short-chain peptide powders prepared by the process of the invention with improved product characteristics.

### Disclosure of the invention

### Short description of the invention

The inventors have found that the above cited problems can be solved by subjecting short-chain peptide solutions to a process synergistically combining the steps of a. flash-freezing droplets of peptide solution to form pellets in a liquefied gas bath and b. controlled lyophilization of the short-chain peptide pellets so obtained.

To this end, the inventors have developed a method for preparing short-chain peptide powder particles comprising the steps of:
a. freezing droplets of a solution containing a short-chain peptide in a solvent to form pellets by contacting the droplets of solution with a liquefied gas and
b. removing the solution solvent and optionally other volatiles, such as e.g. solvent aid and/or residual acids, from the frozen pellets by lyophilization.

Surprisingly, the quality defects associated with the aggregation of such short-chain peptides do not occur when powder particles are prepared through the combined use of the above process steps. Moreover, it has been surprisingly found that the combined use of the above process steps reproducibly and without loss of quality leads to short-chain peptide powders with improved specifications in terms of short-chain peptide content, residual solvent content, residual acid content and/or counter-ion content.

### Preferred modes of the invention

In a preferred process according to the invention, the droplets are formed by passing the solution through nozzles, and/or the liquefied gas is liquid nitrogen and/or argon.

In a preferred process according to the invention, the short-chain peptide contains equal to or less than 100, preferably equal to or less than 50, more preferred equal to or less than 30, even more preferred equal to or less than 20 and most preferred equal to or less than 10 amino acids.

In a further preferred mode of the invention, the average diameter of the frozen pellets resulting from the first process step is from 1 to 6 mm, more preferably from 3 to 5 mm.

In a further preferred mode of the invention, the concentration of short-chain peptide in the solution ranges from 3.5 to 100, more preferably from 10 to 50 g/liter.

In a further preferred mode of the invention, the solution containing the short-chain peptides is devoid of additives and/or optionally contains an acid compound. The term "devoid" is intended to denote a concentration (weight %) of the additive and/or acid equal to or less than 1 %, more preferably equal to or less than 0.1 %, even more preferably equal to or less than 0.01 %.

In a further preferred mode of the invention, the lyophilization step comprises a two-stage process, wherein the vacuum during the second stage of the lyophilization is substantially higher compared to the first and/or the duration of the second stage of the lyophilization is usually one third of the overall lyophilization duration.

In a further preferred mode of the invention, the flash-freezing step comprises the use of pure-grade liquid nitrogen devoid of impurities. The term "devoid" is intended to denote a concentration (weight %) of the additive and/or acid equal to or less than 1 %, more preferably equal to or less than 0.1 %, even more preferably equal to or less than 0.01 %.

In a preferred mode of the invention, the short-chain peptides are synthetic short-chain peptides. The term "synthetic short-chain peptides" is intended to denote that the peptides are obtained by chemical reaction between different amino acid derivatives ; any pathway or process known in the art for peptide synthesis, like processes in liquid or solution phase, or on a solid support (solid-phase synthesis) may be applied. In a more preferred mode of the invention, at least one amino acid in the synthetic short-chain peptide comprises a positively chargeable side chain, even more preferably the amino acid comprising the positively chargeable side chain is arginine, histidine, or lysine, most preferably arginine.

In a preferred mode of the invention short-chain peptide powder is provided wherein the concentration (weight %) of a counter ion, such as e.g. acetate or trifluoroacetate, is less than 20 %, more preferably less than 15 %, even more preferably equal to or less than 14 %.

Further preferred embodiments of the invention are set forth in the dependent claims.

### Detailed description of the invention

### Short-chain peptides

For the purpose of the present invention, the term "amino acid" is intended to denote any compound comprising at least one -NR1R2 group, wherein R1 and R2 independently denote hydrogen or a C1 to C6 branched or unbranched alkyl group, preferably hydrogen, and comprising at least one carboxyl group. The term "peptide" refers to a polymer in which the monomers are amino acids covalently attached to each other by means of amide bonds.

### Flash-freezing to form pellets

In general terms, the method or process according to the present invention comprises two steps that proceed in sequential order, first flash-freezing, followed by lyophilization. These combined steps result in a morphologically controlled short-chain peptide in powder form.

Flash-freezing is a technique whereby the solution enters a batch pelletizer through a dispenser, comprising various nozzles, in the form of uniform droplets.

The batch pelletizer comprises an insulated container filled with liquid nitrogen, stirred through the injection of nitrogen gas, creating a vortex in the batch. The liquid nitrogen temperature is held at -196°C.

The uniform droplets of peptide solution, upon contact with the stirred liquid nitrogen, immediately freeze - hence the term flash-freezing - and remain in the container until the end of the process.

The immediate freezing results from the inherent high ratio of surface to volume of the small droplets, contrary to the situation of the prior art freezing of a big volume of peptide solution. For example, according to the process of our invention, a time of only 15-20 seconds is required to obtain fully frozen pellets. This immediate freezing has the beneficial effect of keeping the peptide solution more or less in its amorphous state, and hence enables full control of the morphology of the short-chain peptides. Negative effects of possible chemical transformations and product concentrations are reduced to an extent that the resulting product quality remains indeed fully intact. The presence of acids has been reported in the solution of peptides to be lyophilized. With the traditional way of lyophilization, water is extracted from the system by the formation of pure water crystals. This phenomenon raises the concentration of the peptide in the liquid water phase, which might give rise to a change in pH, and the generation of structural heterogeneousness.

Although other freezing agents can be envisaged in the process according to the invention, liquid nitrogen or liquid argon is preferred, and liquid nitrogen is more preferred. Products can then be frozen at unmatched controlled freezing rates down below any critical temperature minimizing the formation of crystalline ice during freezing. When the traditional freezing process of the short-chain peptide solution is used, the peptides tend to present a glassy state due to the pure water ice crystals formation during the slow freezing process, causing problems such as aggregation as the concentration of peptides in the peptide solution tends to increase due to the growth and the quantity of pure water ice crystals formation during the too slow cooling. Since the cooling occurring as a result of the flash-freezing step of the process of our invention is markedly quicker, this phenomenon does not occur, and hence higher short-chain peptide concentrations can be used.

In the process according to the invention, the concentration of the short-chain peptides in the solution should preferably be from 3.5 to 100 g/liter, more preferably from 10 to 50 g/liter.

The concentration of the short-chain peptide in the solution is generally a function of the solubility of the peptide to be subjected to the process according to the invention. The solution should remain stable during filtration after synthesis and/or purification and retain such stability throughout the entire process cycle.

In most cases the peptide solution is prepared, resp. kept in an aqueous solvent at room temperature or in a chill room. So the temperature of the peptide solution is generally kept in a temperature range from 4 to 35°C.

In order to increase the solubility, and avoid aggregation during the various stages of the process, additives such as a solvent aid can be used. Examples for solvent aids are 2,2,2-trifluoroethanol, dimethylsulfoxide, acetonitrile and terbutanol.

Such solvent aids are however difficult to separate from the short-chain peptide during lyophilization.

According to a preferred mode of operation of our invention however, no additives are used, so as to obtain pure peptide powders, devoid of any additives.

During flash-freezing direct contact between the peptide solution and the liquid nitrogen occurs. This might give rise to contamination of the short-chain peptides with impurities in the liquid nitrogen, which should be avoided. To this end pure nitrogen, without impurities, or alternatively with impurities under a critical level, should be used during the flash freezing process. These levels should be in accordance with the process restrictions imposed by Good Manufacturing Practices (GMP).

As the pellet formation is a critical step of the process, care should be taken to the following operational parameters :
Cryo-equipment : To avoid contamination of the short-chain peptides, stainless steel sufficiently polished to avoid e.g. bacteria sticking to its walls should be used.
Size of the dispenser nozzles : It should be pointed out that, contrary to the flash-freezing technique as applied to proteins, there is no need to make use of extremely small nozzle sizes resulting in an atomizing effect of the protein solution and thus, extremely small droplets.

On the contrary, in view of the efficiency of the overall operation, the size of the nozzles may be designed such that the resulting droplets have an average diameter from 1 to 6 mm, preferably from 3 to 5 mm.
Size of the pellets : Contrarily to the extremely small pellets resulting from the small nozzles in the prior art, the efficiency of the overall operation of the present invention allows the pellets to have an average diameter from 1 to 6 mm, preferably from 3 to 5 mm. Pellet sizes below 1 mm give rise to difficulties in handling too small frozen pellets in the subsequent steps of the procedure. To the extent aggregation is an issue for short-chain peptides, it can be effectively countered by pellets of relatively larger size as mentioned above. On the other hand, the upper limit of 6 mm should be chosen to assure that the droplets are effectively frozen.

In order to illustrate the above, a frozen pellet with an average diameter of 5 mm, for a solution of peptide containing 10 g of peptide per liter, yields a dry powder particle of around 0.65 mg.

The same concentration of solution, 10 g/liter, for a frozen pellet with an average diameter of 1 mm, will yield a dry powder particle of around 0.005 mg.

Hence, the limitations as set forth above in terms of diameters for the frozen pellets.

Upon completion of this first process step, the frozen pellets are optionally removed from the container and subjected to the lyophilization step.

### Lyophilization step

In principle, in the second step of the process according to the invention - the lyophilization step - the primary aim is to preserve or keep the advantages obtained in the first step of the process, whilst removing the solvent and optionally other volatiles, such as e.g. solvent aids and/or residual acids, present in the pellets. In most practical applications the solvent is water. In this step, the solvent and optionally other volatiles are removed by sublimation, occurring under relative vacuum, specified temperature ranges and durations.

To this end, care must indeed be taken that the sublimation process to evacuate the solvent - in most applications water - and optionally other volatiles should take place under quite controlled conditions of pressure or relative vacuum, shelves temperature, as well as duration of the process.

To this end, pressure increase or loss of relative vacuum can be used as a means to control the process.

Overall, the process according to our invention preferably comprises a lyophilization step comprising two stages, a so-called primary drying stage and a secondary drying stage.

In one aspect of the invention, the vacuum during the secondary drying stage is substantially higher compared to the vacuum applied in the primary drying stage.

Generally speaking, the degrees of vacuum, the duration and the temperature applied during the first and second stages of the lyophilization step generally depend on the kind of short-chain peptide treated and are optimized taking into account the nature of the short-chain peptide treated by the person skilled in the art.

Typical values for the vacuum during the primary drying stage range from 1 to 0.2 mbar, and during the secondary drying stage from 0.5mbar to 0.01 mbar.

Typical temperature ranges used during the primary drying stage range from -80°C to +20°C or alternatively from -80°C to +30°C. Typical temperature ranges used during the secondary drying stage range from +20°C to +60°C.

### Examples

In order to illustrate the beneficial effects of the process according to the present invention, samples were subjected to a traditional freezing and lyophilization process of the prior art, and the results were compared to the combined flash-freezing and lyophilization process according to the present invention.

The samples contained an octapeptide comprising one protonated arginine moiety and a counter ion in form of acetate.

Samples were prepared with a concentration of peptide in the solution of 20, resp. 40 g/liter.

In the traditional freezing cycle, as illustrated by the comparative examples, the filling height of the liquid peptide solution in GORE™ LYOGUARD^{®} Freeze-Drying Containers was approx. 10 mm.
**Column 1** in the table below indicates the sample number ; the first 4 numbers are the comparative examples, using the traditional freezing, whereas numbers 5 to 8 refer to the samples prepared according to our invention.
**Column 2** indicates the short-chain peptide concentration of the solution of the sample, expressed in g/liter, subjected to traditional flash freezing, resp. flash freezing according to our invention.
**Column 3** in the table below indicates the amount of short-chain peptide in the final sample as produced ; this amount was determined on the basis of NMR (Nuclear Magnetic Resonance) analysis, and expressed in weight % of the total sample content.
**Column 4** indicates the amount of acetate, also as determined by NMR, and also expressed as a weight % of the total sample weight.
**Column 5** indicates the water content of the samples as produced, determined by Karl Fisher methodology.
The sum of the values of columns 3, 4 and 5 should therefore equal 100 %, apart from deviations inherently linked to the accuracy of the analysis methods used.
**Column 6** finally indicates the amount of pure short-chain peptide in the final peptide powder, as determined by HPLC (High Performance Liquid Chromatography). This analysis is performed on the whole of the peptides resulting from the analysis method of column 3.

| **Column** | **Column** | **Column** | **Column** | **Column** | **Column** |
|---|---|---|---|---|---|
| **1** | **2** | **3** | **4** | **5** | **6** |
| **Test Nr.** | **Peptide** | **Pep.** | **Acetate** | **Water** | **Pept.** |
| | **Conc.** | **Am. %** | **Am. %** | **Cont. %** | **Conc. %** |
| *Traditional Freeze-Drying* | | | | | |
| *Drying program A* | | | | | |
| Comp. 1 | 20 | 83 | 16 | 1.23 | 95.9 |
| Comp. 3 | 40 | 78 | 17 | 2.07 | 96.0 |
| *Drying Program B* | | | | | |
| Comp. 2 | 20 | 79 | 15 | 1.48 | 95.9 |
| Comp. 4 | 40 | 80 | 16 | 2.44 | 95.9 |
| *Flash-Freezing and Lyophilization* | | | | | |
| *Drying Program A* | | | | | |
| Ex. 5 | 20 | 84 | 13 | 1.34 | 95.9 |
| Ex. 7 | 40 | 83 | 13 | 2.43 | 95.8 |
| *Drying Program B* | | | | | |
| Ex. 6 | 20 | 83 | 12 | 1.76 | 95.9 |
| Ex. 8 | 40 | 83 | 12 | 2.42 | 95.8 |

The specifications for Drying Program A, resp. B were as indicated in the below table.

Herein, the time is expressed in hours and minutes, temperature is expressed in °C, vacuum is expressed in mbar.

| | **Drying Program A** | | | **Drying Program B** | | |
|---|---|---|---|---|---|---|
| Process Phase | Time | Temp | Vacuum | Time | Temp | Vacuum |
| 1. Precooling | 00.00 | +20 | 1000 | 00.00 | +20 | 1000 |
| 2. Freezing | 00.30 | -40 | 1000 | 00.30 | -40 | 1000 |
| 3. Freezing | 02.30 | -40 | 1000 | 02.30 | -40 | 1000 |
| 4. Sublimation | 00.05 | -40 | 1 | 00.05 | -40 | 1 |
| 5. Sublimation | 00.10 | -40 | 0.22 | 00.10 | -40 | 0.22 |
| 6. Sublimation | 02.00 | -10 | 0.22 | 02.00 | -15 | 0.22 |
| 7. Sublimation | 20.00 | +0 | 0.22 | 15.00 | +0 | 0.22 |
| 8. Sublimation | 02.00 | +20 | 0.22 | 02.00 | +30 | 0.22 |
| 9. Sublimation | 08.00 | +20 | 0.22 | 08.00 | +30 | 0.22 |
| 10. Second. Drying | 00.40 | +50 | 0.021 | 00.20 | +50 | 0.021 |
| 11. Second. Drying | 10.00 | +50 | 0.021 | 15.00 | +50 | 0.021 |
| Total Process Time | 45.55 | | | 45.35 | | |

From the above table, it is clear that when the process according to our invention is employed, the amount of the counter ion acetate contained in the short-chain peptide product was far below both the critical limit of 20 % and even below the preferred level level of 15 %.

Generally, the amount of acetate in the product produced by the process according to the invention was approximately 3 % lower than the amount of acetate in the comparative examples. In conformity herewith, the peptide amount of the samples prepared according to the method of our invention was approximately 3 % higher compared to the peptide content of the samples produced according to the traditional method.

Additionally, the amount of acetate in the different samples prepared according to the method of our invention was within narrow margins, ranging from 12 to 13 %, whereas in the samples prepared according to the traditional method, the acetate content varied from 15 up to 17 %.

Generally spoken, the process of our invention enables to reach the set specifications in terms of the content of counter ion, such as e.g. acetate or trifluoroacetate, in the short chain peptide powders in a more efficient manner. The process also enables to reach such specifications in a more homogeneous manner, i.e. with less variation between the various production batches.

The above not only holds for the specifications in terms of the counter ion, such as e.g. acetate or trifluoroacetate, but equally well in terms of the other remaining specifications of the short-chain peptide powders, e.g. the residual solvent (water) content, and/or the residual solvent aid content, in case such solvent aids, like e.g. acetonitrile or terbutanol are used.

The latter is an indication not only of the superior quality of the peptide powders prepared following the process according to our invention, but also of the enhanced reproducibility of such quality when the process according to our invention is employed.

On the other hand, the peptide powdery samples prepared according to the process of our invention all showed a homogeneous pattern, and no aggregation.

Finally, the above table also illustrates that when the process of our invention is used, even in the case when the original starting solution of peptides is characterized by a higher concentration of peptides (e.g. 40 g/liter instead of 20 g/liter), the amount of peptides as determined by NMR in the final powdery samples remain. Indeed in all four examples 5 to 8, the concentration of peptides is 83-84 %, whereas in the comparative examples, this concentration is only 80 % at most when concentrated (40 g/l) peptide solutions are used (comp. ex. 3 and 4).

## Claims

1. A method for preparing a short-chain peptide powder comprising the steps of
a. flash-freezing droplets of a solution comprising a short-chain peptide and a solvent to form pellets by contacting the droplets with a liquefied gas, wherein the droplets are formed by passing the solution through nozzles and
b. lyophilization of the so formed pellets,
wherein the average diameter of the frozen pellets resulting from the process step a. is from 1 to 6 mm.

2. The method of claim 1, wherein the liquefied gas is liquid nitrogen or liquid argon, preferably liquid nitrogen.

3. The method of any of the preceding claims, wherein the short-chain peptide contains equal to or less than 100, preferably equal to or less than 50, more preferably equal to or less than 30, even more preferably equal to or less than 20 and most preferred equal to or less than 10 amino acids.

4. The method of any of the preceding claims, wherein the average diameter of the frozen pellets resulting from the first process step a. is from 3 to 5 mm.

5. The method of any of the preceding claims, wherein the concentration of the short-chain peptide in the solution is from 3.5 to 100 g/liter, preferably from 10 to 50 g/liter.

6. The method of any of the preceding claims, wherein the solution containing the short-chain peptide is devoid of additives.

7. The method of any of the preceding claims, wherein the solution of the short-chain peptide contains an acid compound.

8. The method according to any of the preceding claims, wherein the flash-freezing step a. comprises the use of pure-grade liquid nitrogen devoid of impurities.

9. The method of any of the preceding claims, wherein the lyophilization step b. comprises a two-stage process, wherein the degree of vacuum during the second stage of the lyophilization is substantially higher compared to the degree of vacuum during the first stage, wherein the vacuum during the first drying stage ranges from 1 to 0.2 mbar and during the second drying stage from 0.5 mbar to 0.01 mbar.

10. A short-chain peptide powder prepared by the method of any one of the preceding claims **characterised in that** at least one amino acid in the short-chain peptide comprises a positively chargeable side-chain.

11. A short-chain peptide powder according to claim 10 **characterised in that** the short-chain peptide powder comprises a counter-ion, preferably acetate or trifluoroacetate.

12. A short-chain peptide powder according to claim 10 or 11 **characterised in that** the chemical purity of the short-chain peptide is equal to or higher than 95 % and/or the content in weight % of the counter-ion is less than 15 %.

13. Pellets obtainable by the method of claim 1, wherein the pellets are prepared by flash-freezing droplets of a solution comprising a short-chain peptide and a solvent by contacting the droplets with a liquefied gas and wherein the said pellets have an average diameter from 1 to 6 mm.

14. Pellets according to claim 13 **characterised in that** equal to or less than 10 % of the short-chain peptide shows agglomeration, preferably equal to or less than 5 % of the short-chain peptide shows agglomeration.

## Patentansprüche

1. Verfahren zur Herstellung eines Kurzkettenpeptidpulvers, umfassend die Schritte:
a. Schockgefrieren von Tröpfchen einer Lösung, umfassend ein Kurzkettenpeptid und ein Lösungsmittel, zum Bilden von Pellets, indem die Tröpfchen mit einem verflüssigten Gas in Kontakt gebracht werden,
wobei die Tröpfchen gebildet werden, indem die Lösung durch Düsen geführt wird, und
b. Lyophilisierung der so gebildeten Pellets,
wobei der mittlere Durchmesser der gefrorenen Pellets, die sich aus dem Prozessschritt a. ergeben, 1 bis 6 mm beträgt.

2. Verfahren nach Anspruch 1, wobei das verflüssigte Gas Flüssigstickstoff oder Flüssigargon ist, vorzugsweise Flüssigstickstoff.

3. Verfahren nach einem der vorhergehenden Ansprüche, wobei das Kurzkettenpeptid gleich oder weniger als 100, vorzugsweise gleich oder weniger als 50, noch bevorzugter gleich oder weniger als 30, sogar noch bevorzugter gleich oder weniger als 20 und am bevorzugtesten gleich oder weniger als 10 Aminosäuren enthält.

4. Verfahren nach einem der vorhergehenden Ansprüche, wobei der mittlere Durchmesser der gefrorenen Pellets, die sich aus dem Prozessschritt a. ergeben, 3 bis 5 mm beträgt.

5. Verfahren nach einem der vorhergehenden Ansprüche, wobei die Konzentration des Kurzkettenpeptids in der Lösung 3,5 bis 100 g/Liter beträgt, vorzugsweise 10 bis 50 g/Liter.

6. Verfahren nach einem der vorhergehenden Ansprüche, wobei die Lösung, die das Kurzkettenpeptid enthält, frei von Zusätzen ist.

7. Verfahren nach einem der vorhergehenden Ansprüche, wobei die Lösung des Kurzkettenpeptids eine Säureverbindung enthält.

8. Verfahren nach einem der vorhergehenden Ansprüche, wobei der Schritt a. des Schockgefrierens die Verwendung von reinem Flüssigstickstoff frei von Fremdstoffen umfasst.

9. Verfahren nach einem der vorhergehenden Ansprüche, wobei der Schritt b. der Lyophilisierung einen zweistufigen Prozess umfasst, wobei der Vakuumsgrad während der zweiten Stufe der Lyophilisierung verglichen mit dem Vakuumsgrad während der ersten Stufe wesentlich höher ist, wobei das Vakuum während der ersten Trocknungsstufe von 1 bis 0,2 mbar und während der zweiten Stufe von 0,5 mbar bis 0,01 mbar beträgt.

10. Kurzkettenpeptidpulver, zubereitet durch das Verfahren nach einem der vorhergehenden Ansprüche, **dadurch gekennzeichnet, dass** mindestens eine Aminosäure im Kurzkettenpeptid eine positiv ladbare Seitenkette aufweist.

11. Kurzkettenpeptidpulver nach Anspruch 10, **dadurch gekennzeichnet, dass** das Kurzkettenpeptidpulver ein Gegenion umfasst, vorzugsweise Acetat oder Trifluoracetat.

12. Kurzkettenpeptidpulver nach Anspruch 10 oder 11, **dadurch gekennzeichnet, dass** die chemische Reinheit des Kurzkettenpeptids gleich oder höher als 90 % ist und/oder der Gegeniongehalt in Gewichts-% weniger als 15 % beträgt.

13. Pellets, erzielbar durch das Verfahren nach Anspruch 1, wobei die Pellets durch Schockgefrieren von Tröpfchen einer Lösung, umfassend ein Kurzkettenpeptid und ein Lösungsmittel, zubereitet werden, indem die Tröpfchen mit einem verflüssigten Gas in Kontakt gebracht werden, und wobei die Pellets einen mittleren Durchmesser von 1 bis 6 mm aufweisen.

14. Pellets nach Anspruch 13, **dadurch gekennzeichnet, dass** gleich oder weniger als 10 % des Kurzkettenpeptids eine Agglomeration zeigen, vorzugsweise gleich oder weniger als 5 % des Kurzkettenpeptids eine Agglomeration zeigen.

## Revendications

1. Une méthode de préparation d'une poudre de peptide ayant une courte chaine comprenant les étapes de :
a. congélation flash de gouttelettes d'une solution comprenant un peptide de courte chaine et un solvant pour former des pellets en mettant en contact les gouttelettes avec un gaz liquéfié, dans laquelle les gouttelettes sont formées en faisant passer la solution à travers un brumatiseur et
b. lyophilisation des pellets ainsi formés,
dans laquelle le diamètre moyen des pellets congelés résultant de l'étape a. du procédé est de 1 à 6 mm.

2. La méthode de la revendication 1, dans laquelle le gaz liquéfié est l'azote liquide ou l'argon liquide, de préférence l'azote liquide.

3. La méthode d'une quelconque des revendications précédentes, dans laquelle le peptide de courte chaine contient 100 ou moins que 100, de préférence 50 ou moins que 50, de manière plus préférée 30 ou moins que 30, encore plus préféré 20 ou moins que 20 et de manière la plus préférée 10 ou moins d'acides aminés.

4. La méthode d'une quelconque des revendications précédentes, dans laquelle le diamètre moyen des pellets congelés résultants de la première étape du procédé a. est de 3 à 5 mm.

5. La méthode d'une quelconque des revendications précédentes, dans laquelle la concentration du peptide de courte chaine dans la solution est de 3,5 à 100 g/litre, de préférence de 10 à 50 g/litre.

6. La méthode d'une quelconque des revendications précédentes, dans laquelle la solution contenant le peptide de courte chaine est dépourvue d'additifs.

7. La méthode d'une quelconque des revendications précédentes, dans laquelle la solution du peptide de courte chaine contient un composant acide.

8. La méthode d'une quelconque des revendications précédentes, dans laquelle l'étape de congélation flash a. comprend l'utilisation d'azote liquide pur dépourvu d'impuretés.

9. La méthode d'une quelconque des revendications précédentes, dans laquelle l'étape de lyophilisation b. comprend un procédé en deux étapes, dans lequel le degré de vide au cours de la seconde étape de lyophilisation est substantiellement supérieur en comparaison du degré de vide pendant la première étape, dans laquelle le degré de vide pendant la première étape de séchage varie de 1 à 0,2 mbar et pendant la seconde étape de séchage de 0,5 mbar à 0,01 mbar.

10. Une poudre de peptide de courte chaine préparée par la méthode d'une quelconque des revendications précédentes **caractérisée en ce que** au moins un acide aminé du peptide de courte chaine comprend une chaine latérale qui puisse être chargée positivement.

11. Une poudre de peptide de courte chaine selon la revendication 10 **caractérisée en ce que** la poudre de peptide à courte chaine comprend un contre ion, de préférence l'acétate ou le trifluoroacétate.

12. Une poudre de peptide de courte chaine selon la revendication 10 ou 11 **caractérisée en ce que** la pureté chimique du peptide de courte chaine est égale ou supérieure à 95% et/ou la teneur en % pondéraux du contre ion est de moins que 15%.

13. Pellets susceptibles d'être obtenus par la méthode de la revendication 1, dans lesquels les pellets sont préparés par congélation flash de gouttelettes d'une solution comprenant un peptide de courte chaine et un solvant en mettant les gouttelettes au contact avec un gaz liquéfié et dans lesquels les dits pellets ont un diamètre moyen de 1 à 6 mm.

14. Pellets selon la revendication 13 **caractérisés en ce que** 10% ou moins du peptide de courte chaine présente une agglomération, de préférence 5% ou moins du peptide de courte chaine présente une agglomération.
